# EUROPEAN PATENT SPECIFICATION

(11) **EP 3 068 373 B1**
(45) Date of publication and mention of the grant of the patent: **23.06.2021**
(21) Application number: 14816464.3
(22) Date of filing: 12.11.2014
(51) Int. Cl.: A61K 9/00, A61K 45/06, A61K 47/36, A61K 31/165, A61K 31/045, A61K 31/05, A61K 31/085, A61K 31/167, A61K 31/728, A61P 19/02, A61P 25/04, A61P 29/00, A61P 43/00, A61P 17/02

(54) **AQUEOUS BASED CAPSAICINOID FORMULATIONS AND METHODS OF MANUFACTURE AND USE**
WASSERBASIERTE CAPSAICINOIDFORMULIERUNGEN UND VERFAHREN ZUR HERSTELLUNG UND VERWENDUNG
FORMULATIONS DE CAPSAICINOÏDE À BASE AQUEUSE ET PROCÉDÉS DE FABRICATION ET D'UTILISATION

(30) Priority: 12.11.2013 US 201314078253; 15.04.2014 US 201414253660
(43) Date of publication of application: 21.09.2016
(73) Proprietor: Propella Therapeutics, Inc., Pittsboro NC 27312 (US)
(72) Inventor: BIRBARA, Charles A., Worcester, MA 01609 (US); BIRBARA, Philip J., West Hartford, CT 06119 (US); BUCKS, Daniel, Millbrae, CA 94030 (US); COUGHLIN, Mary, Worcester, MA 01606 (US)
(74) Representative: Stafford, Jonathan Alan Lewis
(86) International application number: PCT/US2014/065290
(87) International publication number: WO 2015/073577

(56) References cited:
- WO-A2-2006/058140
- WO-A2-2014/075084
- US-A1- 2011 311 592
- None

## Description

### Field of the Invention

The present invention is directed to compositions for the administration of a capsaicinoid into localized areas. The compositions are useful for the treatment of multiple disorders involving pain (acute and chronic, moderate to severe), and they provide extended pain relief. The disclosure also relates to methods including pretreatments which limit burning and stinging pain associated with capsaicinoid injection.

### Background of the Invention

Capsaicin, a pungent substance in the fruit of capsicum plants, works to relieve pain by causing a localized degradation of the C neuron endings; capsaicinoids being the only analgesics known to relieve pain by this mechanism. The activity of capsaicin results from its binding to, and activating, an ion channel called vanilloid receptor 1 (VR1). Under normal circumstances, when the VR1 ion channel is activated it opens for a short time, causing the C neurons to transmit a pain signal toward the brain. When capsaicin binds to, and activates VR1, it causes a series of events within the cell that degrade the C neural endings, or terminals of the C neuron, thereby preventing the neuron from transmitting pain signals.

Although capsaicin's analgesic effect was thought to be due to depletion of the substance P, recent evidence suggests a process of "defunctionalization" of nociceptor fibers as being responsible for the analgesic effect of capsaicin. *(*Anand P, Bley K. Topical capsaicin for pain management: therapeutic potential and mechanisms of action of the new high-concentration capsaicin 8% patch. Br J Anaesth. 2011;107(4):490-502*.)*

Capsaicin topical ointments and creams relieve minor aches and pains of muscles and joints. Capsaicin is currently marketed as over-the-counter topically applied, non-sterile creams and patches containing capsaicin at low doses. Concentrations of capsaicin are typically between 0.025 wt. % and 0.075 wt. %. These over-the-counter topical preparations are used topically by consumers to relieve pain with variable and often inadequate results when used to treat conditions such as osteoarthritis, shingles (herpes zoster), psoriasis and diabetic neuropathy. Capsaicin is also available in large adhesive bandages that can be applied to the back.

Topical preparations of capsaicin are used in a variety of skin disorders that involve pain and itching, such as post herpetic neuralgia, diabetic neuropathy, prorates, psoriasis, cluster headache, post mastectomy pain syndrome, rhinopathy, oral mucositis, cutaneous allergy, detrusor hyperreflexia, loin pain/hematuria syndrome, neck pain, amputation stump pain, reflex sympathetic dystrophy, skin tumor, arthritis including rheumatoid arthritis and osteoarthritis, post-surgical pain, oral pain, and pain caused by injury, amongst others. *(*Martin Hautkappe et al., Review of the Effectiveness of Capsaicin for Painful Cutaneous Disorders and Neural Dysfunction, Clin. J. Pain, 14:97-106, 1998*).*

Hyaluronic acid (HA) or hyaluronan is a glycosaminoglycan constituent of synovial fluid. HA is responsible for the viscoelastic quality of synovial fluid that acts as both a lubricant and shock absorber. Injection of HA preparations into the knee and hip is commonly used to treat osteoarthritis, but there is debate over the efficacy of the treatment and benefit-to-risk ratio. A Cochrane review of 40 placebo-controlled trials with five different hyaluronan products found statistically significant improvements in pain on weight bearing when results were pooled, but improvements were variable.

Injections of hyaluronic acid (such as Supartz, Hyalgan, Synvisc, Artzal, and Nuflexxa) into the joint -- a procedure called viscosupplementation - can provide pain relief for knee osteoarthritis. No major safety issues were detected, but in placebo-controlled trials minor adverse events such as transient pain at the injection site occurred slightly more frequently in patients treated with intra-articular hyaluronan than in those treated with intra-articular corticosteroids. *(*Zhang et al,. OARSI recommendations for the management of hip and knee osteoarthritis, part II: OARSI evidence-based, expert consensus guidelines. Osteoarthritis Cartilage 2008; 16:137-162*.)*

Further, HA has been shown to induce analgesia in a bradykinin-induced model of joint pain in rats. This analgesic action was also molecular weight (MW)-dependent, as significant effects were observed at lower concentrations with a higher-MW formulation than with lower-MW HAs. *(*Gotoh Set al: Effects of the molecular weight of hyaluronic acid and its action mechanisms on experimental jointpain in rats. Ann Rheum Dis 1993, 52:817-822*).*

HA may have direct or indirect effects on substance P, which can be involved in pain. Since substance P interacts with excitatory amino acids, prostaglandins, and NO, the effects of HA on these factors can indirectly affect the pharmacology of substance P. Additionally, HA has been shown to inhibit an increased vascular permeability induced by substance P.
*(*Moore et al: Hyaluronan as a drug delivery system for diclofenac: a hypothesis for mode of action. Int J Tissue React 1995, 17:153-156*.)*

Anti-inflammatories decrease the inflammation and swelling at the tissue site. They do this by blocking certain physiologically active substances. For example, these substances, prostaglandin, substance P and histamine, can cause small arteries to dilate with subsequent edema or fluid formation. Soft tissue, muscle, and nerve cells become irritable inflamed and hyper excitable. Anti-inflammatory medications can inhibit this inflammatory process. The treatment of chronic pain with anti-inflammatories however, is limited.

The mainstays for pain relief after total hip arthroplasty and total knee arthroplasty have been the opioids. Although these medications are excellent analgesics, opioids have problems that limit their effectiveness. Alternative analgesics have been considered too mild for the pain caused by these procedures.

US Patent 5962532 discloses methods and compositions for treating pain at a specific site with an effective concentration of capsaicin or analogues thereof. The methods involve providing anesthesia to the site where the capsaicin or analogues thereof is to be administered, and then administering an effective concentration of capsaicin to the joint.

US Patent 8,158,682 relates to methods for treating or attenuating pain in a patient. Specifically, the disclosure provides a method for attenuating pain in proximity to the site of an open wound or surgical incision comprising instilling a pharmaceutical composition comprising a capsaicinoid into the wound or incision, allowing the pharmaceutical composition to dwell for a predetermined period of time, and aspirating the wound or incision to remove the pharmaceutical composition. The disclosure also provides a method for attenuating pain in proximity to a joint comprising intra-articularly injecting a pharmaceutical composition comprising a capsaicinoid into the joint, allowing the pharmaceutical composition to dwell for predetermined period of time, and aspirating the joint to remove the pharmaceutical composition. In certain embodiments of the disclosure, the capsaicinoid is capsaicin.

US Patent 8,420,600 discloses compositions and methods for relieving pain at a site in a human or animal by administering at a discrete site in a human or animal a dose of capsaicin in an amount effective to denervate the discrete site without eliciting an effect outside the discrete location.

US 2004/0161481 discloses a method for relieving pain at a site in a human or animal in need thereof, comprising administering by injection or infiltration, a dose of a capsaicinoid and coadministering an effective amount of a NSAID to decrease an undesired effect of the capsaicinoid.

US 2004/0156931 discloses a method for relieving pain at a site in a human or animal in need thereof, comprising administering by injection or infiltration, a dose of a capsaicinoid and co administering a vasodilator.

US 2004/0186182 discloses a method for relieving pain at a site in a human or animal in need thereof, comprising administering by injection or infiltration, a dose of a capsaicinoid and co administering a non-anesthetic sodium channel blocker.

US 2005/0019436 discloses compositions and methods for relieving pain at a site in a human or animal in need thereof by administering at a discrete site in a human or animal in need thereof a dose of capsaicin in an amount effective to denervate a discrete site without eliciting an effect outside the discrete location, the dose of capsaicin ranging from 1 µg to 3000 µg.

US 2005/0020690 discloses compositions and methods for attenuating or relieving pain at a site in a human or animal in need thereof by infiltrating at a surgical site or open wound in a human or animal a dose of capsaicinoid in an amount effective to denervate the surgical site or open wound substantially without eliciting an effect outside the surgical site or open wound.

US 2005/0058734 discloses a method for relieving pain at a site in a human or animal in need thereof, comprising administering by injection or infiltration, a dose of a capsaicinoid and co administering a vasoconstrictor.

US 2006/0269628 discloses compositions and methods for attenuating or relieving pain at a site in a human or animal in need thereof by infiltrating at a surgical site or open wound in a human or animal a dose of capsaicinoid in an amount effective to denervate the surgical site or open wound substantially without eliciting an effect outside the surgical site or open wound.

U.S. Patent Application 2006/0100272 discloses compositions and methods for the treatment of pain, and neuropathic pain in particular.

US 2006/0148903 and WO 2006/058140 A2 provide capsaicinoid gel formulations and methods for relieving pre- and post-surgical pain at a site in a human or animal by administering at a surgical site in a human or animal in need thereof a dose of capsaicinoid gel in an amount effective to attenuate post-surgical pain at the surgical site, the dose of capsaicin ranging from 100 µg to 10,000 µg.

US 2007/0293703 provides methods for synthesizing the trans isomer of capsaicin and/or capsaicin-like compounds by utilizing a process wherein the trans geometry is set from the beginning of the synthesis reaction and carried through the entire synthesis process.

US 2008/0153780 relates to the use of a vanilloid receptor agonist together with a glycosaminoglycan or proteoglycan for producing an agent for treating pain.

US 2007/0036876 provides compositions and methods for relieving pain at a site in a human or animal in need thereof by administering at a discrete site in a human or animal in need thereof a dose of capsaicin in an amount effective to denervate a discrete site without eliciting an effect outside the discrete location, the dose of capsaicin ranging from 1 µg to 3000 µg.

US 2008/0260791 provides compositions and methods for relieving pain at a site in a human or animal in need thereof by administering at a discrete site in a human or animal in need thereof a dose of capsaicin in an amount effective to denervate a discrete site without eliciting an effect outside the discrete location, the dose of capsaicin ranging from 1 µg to 5000 µg.

US 2008/0262091 A1 provides compositions and methods for attenuating or relieving pain at a site in a human or animal in need thereof by infiltrating at a surgical site or open wound in a human or animal a dose of capsaicinoid in an amount effective to denervate the surgical site or open wound substantially without eliciting an effect outside the surgical site or open wound.

US 2009/0062359 relates to a method for relieving pain at a site in a human or animal in need thereof, comprising administering by injection or infiltration, a dose of a capsaicinoid and co administering a non-anesthetic sodium channel blocker.

US 2009/0054527 discloses a method for relieving pain at a site in a human or animal in need thereof, comprising administering by injection or infiltration, a dose of a capsaicinoid and co administering a vasodilator.

US 2009/0111792 discloses a method for relieving pain at a site in a human or animal in need thereof, comprising administering by injection or infiltration, a dose of a capsaicinoid and co administering a tricyclic antidepressant.

US 2009/0117167 A1 discloses a method for relieving pain at a site in a human or animal in need thereof, comprising administering by injection or infiltration, a dose of a capsaicinoid and co administering a vasoconstrictor.

US 2009/0118242 discloses a method for relieving pain at a site in a human or animal in need thereof, comprising administering by injection or infiltration, a dose of a capsaicinoid and co administering an effective amount of a NSAID to decrease an undesired effect of the capsaicinoid.

US 2011/0311592 teaches methods of increasing solubility of poorly soluble compounds and methods of making and using formulations of such compounds.

WO 2014/075084 A2 discloses capsaicinoid formulations and methods of treatment, which can be utilized to treat/attenuate pain in mammals. Typically, administration is via injection at a discrete site to provide pain relief for an extended period of time.

ALGRX-4975 was in clinical developed to treat the pain associated with osteoarthritis, tendonitis and postsurgical conditions, as well as for neuropathic pain occurring secondary to nerve injury and other chronic pain conditions. By targeting only the C neuron pain fibers, ALGRX-4975 was able to produce significant long-term analgesia. In clinical studies, ALGRX-4975 has provided long-term relief of pain from a single treatment as summarized in TABLE I.

**TABLE I - A SUMMARY OF ALGRX-4975 CLINICAL TRAILS**

| **ALGRX-4975** | | **COMMENTS** |
|---|---|---|
| **Volume (ml)** | **Concentration (mg/ml)** | |
| 4 | 0.25 | ⁽¹⁾Significant pain reduction of mean Visual Analogue Scale (VAS) at 8 hr and 24 hr post unilateral bunionectomy after a single intra-operative instillation. |
| 0.5 | 0.2 | ⁽²⁾Significant reduction in pain of intermetatarsal neuroma at week 1 and 4 compared to placebo |
| 0.5 | 0.2 | ⁽²⁾Lowered pain scores in patients with end-stage OA of the knee waiting for knee replacement |
| 15 | 0.067 | ⁽³⁾During inguinal hernia repair improved analgesia relative to placebo following the first 3-4 days post surgery |

| | | |
|---|---|---|
| ⁽¹⁾Cantilon, M. et al, Preliminary safety, tolerability and efficacy of ALGRX 4975 in Osteoarthritis (OA) of the knee, The Journal of Pain, Vol. 6, March, 2005. ⁽²⁾Diamond, E. et al, ALGRX 4975 reduces pain of intermetatarsal neuroma: preliminary results from a randomized, double-blind, placebo-controlled, phase II multicenter clinical trial, The Journal of Pain, Vol. 7, April, 2006. ⁽³⁾Aasvang, E. et al, The effect of wound instillation of a novel purified capsaicin formulation on postherniotomy pain: a double-blind, randomized, placebo-controlled study, Anesthesia and Analgesia, 2008, 107(1), p.282-291 | | |

For several clinical trials, a high purity trans-capsaicin was supplied in vials containing 5 mL of purified capsaicin at concentrations of 0.5 mg/ml dissolved in PEG-300. The capsaicin /PEG-300 solution was stored at a temperature between 15 °C and 25 °C. Within four hours prior to injection, the capsaicin concentrate was diluted to make a formulation comprising about 20% PEG-300, about 1.5 mg/ml histidine and about 5% sucrose. As noted in Table II, the capsaicin concentrations of the 3 listed examples in US Patent 8,420,600 are 0.002 mg/ml, 0.02 mg/ml and 0.06 mg/ml.

**TABLE II - Injectable Capsaicin Dose Levels**

| **Capsaicin Dose Level (mg)** | **Capsaicin Dose Concentration (mg/ml)** | **Total Volume of Injectable Dose (ml)** |
|---|---|---|
| **0.01** | **0.002** | **5** |
| **0.1** | **0.02** | **5** |
| **0.3** | **0.06** | **5** |

Upon capsaicin injection in clinical trials, spontaneous burning pain and hyperalgesia was experienced immediately and persisted for up to 60 minutes. These undesirable side effects were attributed to intense activation and temporary sensitization of the peripheral nociceptors at the site of capsaicin application. This activation and sensitization occur prior to the desensitization phase. In an attempt to control burning and stinging upon capsaicin injection, local anesthetics were injected and then withdrawn, prior to capsaicin injection.

A present limitation on the use of injectable capsaicin formulations is the likelihood of intense burning and stinging (B&S) pain for up to 1 hour and mild pain for another 1 -2 hours. The expected B&S of the capsaicin dose are believed to be from the intense nociceptor discharge occurring during the excitatory phase before nociceptor desensitization.

Work in many models has shown that capsaicin can activate sensory nerves to induce release of neuropeptides, in particular calcitonin gene-related peptide (CGRP) and the inflammatory substance P (SP)Lundberg et al, Co-existence of substance P and calcitonin gene-related peptide-like immunoreactivities in sensory nerves in relation to cardiovascular and bronchoconstrictor effects of capsaicin. Eur. J. Pharmacol., 108, 315-319, (1985). Martling et al, Calcitonin gene-related peptide and the lung: neuronal coexistence with substance P, release by capsaicin and vasodilatory effect. Regul. Pept., 20, 125-139, (1988).

It has been demonstrated that capsaicin induced edema is insensitive to treatment with the anti-inflammatory steroid, dexamethasone, which is in contrast to other forms of inflammatory edema formation. Newbold et al, An Investigation into the mechanism of capsaicin-induced edema in rabbit skin, Brit. J. of Pharma., 114, 570-577, (1995).

Ferrell et al. (Neuroscience Letters 141:259-261, 1992) demonstrated that intra-articular injection of capsaicin into the rat knee results in the significant reduction in the number of unmyelinated fibres and that this reduction was reversible.

There is an unmet need for injectable capsaicin formulations and pain treatment procedures that minimize the acute burning sensation produced following capsaicin injection. Clinical studies conducted with capsaicinoid injections have shown the ability of capsaicin to reduce pre and postsurgical pain, pain caused by osteoarthritis, tendonitis and other surgical procedures. Pain ailments are most often managed with opioids and NSAIDs and chronic use is often limited by side effects. There is an unmet need for safe and effective therapies to treat chronic pain.

### Objects of the Invention

It is an object of the invention to devise formulations for providing pain relief in mammals via injectable capsaicinoids that overcome the intense burning pain experienced with the administration of a capsaicinoid.

A further object of the subject invention is to provide formulations to ameliorate or prevent the burning or stinging associated with capsaicinoid injection administration.

It is another objective of the invention to provide an optically clear solution containing aqueous and lipophilic ingredients that are totally miscible.

It is another objective of the present invention to provide formulations for extended pain relief without sedation or anesthesia such as nerve, spinal or epidural blocks, for a range of therapeutic applications.

Other objects and advantages will be apparent from a review of the following specification.

### Summary of the Invention

The scope of the present invention is defined by the appended claims.

Disclosed is an aqueous composition for injection in a mammal comprising:
i) 0.0002 % - 0.1% by weight of a capsaicinoid,
ii) 99.9 % - 99.9998% by weight of an aqueous vehicle comprising a solubilizing agent to solubilize said capsaicinoid, and an extended release agent to slow the release of said capsaicinoid from said aqueous composition upon injection of said composition in a mammal,
wherein said aqueous vehicle reduces or eliminates the burning and stinging created by said capsaicinoid upon injection. The capsaicinoid is typically trans-capsaicin. The solubilizing agent can be polyethylene glycol, and the extended release agent is typically hyaluronic acid having an average molecular weight of about 1000 kDaltons. The extended release agent can also be collagen, elastin, and a biodegradable polymer matrix.

The aqueous composition may comprise:
0.005 - 0.05 % by weight capsaicin,
5 - 25% by weight polyethylene glycol, and
0.2 - 1% by weight hyaluronic acid.

The aqueous composition may include an anti-inflammatory agent, an analgesic agent, and/or a surfactant (.002-2% by wt), e.g. the nonionic surfactant polyoxy 40 hydrogenated castor oil, or PS 80. The weight ratio of capsaicinoid to polysorbate 80 can be 1 to 5.

The aqueous composition may comprise:
0.005 - 0.05% by weight capsaicin,
0.025 - 0.15% by weight polysorbate 80 or polyoxy 40 hydrogenated castor oil,
0 - 25 % by weight polyethylene glycol 300 or 400, and
0.2 - 1% by weight hyaluronic acid.

The disclosure also relates to a method for treating pain at a site in a mammal, e.g. human, comprising administering to said site in said mammal a therapeutically effective amount of the aqueous composition of the disclosure. An anesthetic is typically administered prior to the administration of said composition. In one embodiment of the disclosure, during the first minute after administering said capsaicinoid composition, the joint is repeatedly flexed and extended. Further, prior to, during and/or after administering said capsaicinoid composition, cooling means can be applied to the site. The capsaicinoid composition is administered in a pharmaceutically acceptable vehicle in a volume from about 0.1 ml to 25 ml.

The invention also relates to a kit for administering a capsaicinoid by intra articular injection according to claim 13.

### Detailed Description of the Invention

The formulations of the invention provide a long-acting, non-opioid, treatment options for the management of moderate to severe chronic (lasting greater than 3 months) or acute pain. Effective capsaicinoid injection therapies must include treatment modalities that address the burning pain following capsaicinoid administration.

Selective and reversible defunctionalization of sensory neurons in patients with disabling chronic pain conditions by site-specific capsaicin injections is an attractive approach for long lasting (weeks to months) pain relief. However, the treatment of joints etc. with the injection of capsaicin to relieve pain is complicated by the intense burning pain capsaicin elicits upon administration.

The capsaicinoid formulations and methods disclosed herein can be utilized to treat/attenuate pain in mammals typically via injection at a discrete site to provide pain relief for an extended period of time. The formulations are administered in a pharmaceutically acceptable vehicle for infiltration. The methods and formulations further include the administration of an extended release agent, anti-inflammatory, analgesic and/or anesthetic in an amount to attenuate the burning and hyperalgesia effects of capsaicinoid administration. This can be done in conjunction with the application of cooling means, e.g.s. ice packs or gel packs, prior to, during, or subsequent to capsaicinoid injection.

Selective and reversible defunctionalization of sensory neurons by site-specific capsaicin injections provides long-lasting (weeks to months) pain relief in patients with disabling chronic pain conditions with no or minor systemic side effects. The capsaicinoid formulations of the invention alleviate or attenuate pain at the site for a prolonged period of time. With respect to pain associated with arthritic conditions such as osteoarthritis, in certain embodiments, a single unit dose capsaicinoid injection or implantation attenuates pain at the site for at least 3 months, for at least 4 months, 5 months or 6 months. With respect to joint pain, in certain embodiments, a single unit dose capsaicinoid injection or implantation attenuates pain at the site for at least one month, at least 3 months, and for 3 to 6 months, and for periods greater than 6 months, e.g. 6 to 12 months. With respect to post-surgical pain, a single unit dose capsaicinoid injection or implantation attenuates pain at the site for at least one week, and in certain embodiments for at least 1 month.

### Compositions of the Invention

The dose of capsaicinoid is prepared for injection, implantation, infiltration or topical application by being incorporated into a pharmaceutically and physiologically acceptable aqueous vehicle for administration with diminished burning sensation upon application. The present invention is directed to the injectable administration of very small quantities of capsaicin into discrete localized areas for the treatment and lessening of pain. Significant advantages result from milligram and/or fractions of milligram quantities of capsaicin in order to produce therapeutic results through alteration of sensory nerve function (TRPV-1) function in a limited area.

The components of the compositions of the invention are discussed below.

### Capsaicinoids

For a general discussion of capsaicinoids, see US patent application 2008/0262091, and commonly owned US Ser. No. 13/609,100. The term "capsaicinoid" as used herein includes capsaicin, a capsaicinoid other that capsaicin, i.e. dihydrocapsaicin, nordihydrocapsaicin, homodihydrocapsaicin, homocapsaicin, and nonivamide, and a mixture of capsaicin with one or more other capsaicinoids. The amount of drug used being based on a therapeutically dose to a dose of capsaicin. Capsaicinoids are 0.0002-.1 % by wt, advantageously 0.005-0.05 % by wt. of the formulations.

Alternatively, a "capsaicin analogue" such as resiniferatoxin, can be administered in place of part or all of the capsaicinoid. The amount of analogue administered being the therapeutically equivalent dose of capsaicin-see US patent application 2008/0262091. Also disclosed is a TRPV1 agonist other than a capsaicinoid, or capsaicin analogue which is utilized in the formulations and methods of the disclosure.

### Delivery Vehicles

The aqueous pharmaceutical compositions of the invention utilize pharmaceutically acceptable delivery vehicles that are single phase aqueous/water systems composed of pharmaceutically acceptable solvents (such as polyethylene glycol (e.g. PEG 300 and PEG 400), ethanol and a nonionic surfactant, e.g.s. polyoxy 40 hydrogenated castor oil (Cremophor RH40), polysorbate 80 (PS 80)). Hyaluronic acid, as an extended release agent, controls the viscosity of the formulation and aids in formulation stability. A significant advantage of the disclosed aqueous based formulations is that water concentrations can exceed 95 wt. %.

Examples of additional components in the aqueous pharmaceutical vehicles include dextrose (sugar) and/or sodium chloride solutions to adjust osmotic pressure and tonicity, as well as buffering agents to adjust pH. The inclusion of 0.9% NaCl, 0.25% phenol, 0.25% menthol and 5% dextrose in the capsaicin/PS 80 aqueous solution did not result in a cloudy appearance or precipitate formation.

### Extended Release Agents

It has been found that certain compounds, which slow the release of a capsaicinoid from the formulation (causing extended release), have diminished burning and stinging effects. Advantageously, the extended release agent will release said capsaicinoid over a period of greater than 15 minutes, 30 minutes, 1 hour , or greater than 4 hours.The capsaicinoid may be released over a period greater than a week. Typically a higher dose will be released over a longer time period. Compositions according to the present invention contain hyaluronic acid as an extended release agent.

### Hyaluronic Acid

The solubilization of capsaicin together with hyaluronic acid and its salts thereof, a substance that is naturally present in the human body that occurs in various tissues (skin, synovial fluids of joints and connective tissues), contributes to ameliorating the burning and stinging associated with topical and injectable capsaicin formulations. Advantageously, 1% to 2% by weight hyaluronic acid or its salts, is used. The hyaluronic acid molecular weight used in the experimental tests described herein had an average molecular weight ranging from 800 to 1,200 kDaltons. While not wishing to be bound by theory, it is believed that the addition of the hyaluronic acid component to the capsaicin forms a polysaccharide network within the aqueous solution and causes capsaicin to be released more slowly in a controlled manner that results in a lessening of the burning and stinging pain. In one embodiment of the invention, the hyaluronic acid is in the form of a cross-linked hydrogel.

### Collagen and Elastin

Collagen is the main structural protein of the various connective tissues in animals. As the main component of connective tissue, it is the most abundant protein in mammals, making up from 25% to 35% of the whole-body protein content. Collagen, in the form of elongated fibrils, is mostly found in fibrous tissues such as tendons, ligaments and skin, and is also abundant in corneas, cartilage, bones, blood vessels, the gut, and intervertebral discs.

Elastin is a protein in connective tissue that is elastic and allows many tissues in the body to resume their shape after stretching or contracting. Elastin helps skin to return to its original position when it is poked or pinched. Elastin is also an important load-bearing tissue in the bodies of vertebrates and used in places where mechanical energy is required to be stored.

Collagen and/or elastin with a capsaicinoid is advantageous in the treatment of pain including OA pain. The addition of the collagen and/or elastin component to a solubilized capsaicin formulation forms a protein network within the aqueous solution that contributes to a delayed or prolonged release of capsaicin thus contributing to minimizing the burning discomfort from either topical application or injection of capsaicin. Additionally, upon injection with a capsaicinoid these naturally occurring high molecular weight proteins function to control the rate of capsaicin release to the nerves to reduce burning, provide lubrication to the sliding bone surfaces. Addition of hyaluronic acid to capsaicinoid formulations containing either or both these naturally occurring high molecular weight proteins further optimizes tolerability and efficacy. In the compositions of the invention, the collagen or elastin must be in the form of a liquid or gel.

### Bioresorbable Polymer Matrix

A bioresorbable polymer matrix, e.g. a cross-linked oxidized dextran hydrogel, can also be used in the formulations of the invention as the extended release agent. See US Patent 8,435,565.

### Solvents

Polyethylene glycol, referred to as PEG, is used as an inactive ingredient, as a solvent, plasticizer, ointment and suppository base, and in tablets and capsules as a lubricant. PEG has low systemic toxicity with systemic absorption less than 0.5%.The term "PEG" is used, in combination with a number. Within the pharmaceutical industry, the number indicates the mean molecular weight. The low-molecular weight liquid polyethylene glycols PEG 300 and 400 are excellent solvents and cosolvents for a large number of substances that do not readily dissolve in water. They are therefore widely used as solvents and solubilizing agents for active substances and excipients in liquid and semi-solid preparations. The ability of PEGs to form complexes with active substances is responsible for their excellent solvent power. Polyethylene glycols can also be used to adjust the viscosity of liquid pharmaceutical preparations and to modify their absorption properties and to stabilize the preparations.

Polyethylene glycols with a mean molecular weight up to 400 are non-volatile liquids at room temperature. Liquid PEGs up to PEG 600 are miscible with water in any ratio. But even higher molecular weight solid PEG grades have excellent solubility in water.

The polyethylene glycols show outstanding toxicological safety regarding acute and chronic oral toxicity, embryotoxicity or skin compatibility, supported by parenteral/ absorption/ excretion investigations. They have been used for many years in cosmetics, foodstuffs and the pharmaceutical industries. Many of these compounds are listed on the FDA Inactive Ingredient List for use in prescription products. Formulations of the disclosure include up to 25% by wt solvents, typically 5-25% by wt.

An alternative to the PEG compounds is polypropylene glycol. Additionally, alcohols including ethyl alcohol, glycerol, polyethylene glycols, etc. can be added to the formulations as capsaicinoid solubilizing agents.

### Surfactants

A surfactant, such as a nonionic surfactant, e.g. PS 80 and/or Cremophor® RH 40 (polyoxyl 40 hydrogenated castor oil); alternatively, Cremophor® ELP, or Solute® HS 15, can be utilized in the formulations of the invention along with a solvent (as described above) or as an alternative to a solvent. The surfactant serves as a wetting agent and emulsifier, and can lessen the initial stinging or burning discomfort associated with capsaicinoid administration.

Further, surfactant/capsaicin (or other capsaicinoids) concentrates can be formed for use in the formulations and methods of the invention as described in commonly owned U.S. Patent 8,637,569. These concentrates can be utilized in the formulations of the invention.

Formulations of the invention may include .001-2.5% by wt surfactants, typically 0.1-0.5 % by wt.

### Analgesic and Anesthetic Agents

Analgesic ingredients are used in the formulations of the invention to ameliorate or prevent the initial acute burning or stinging pain associated with capsaicin. A variety of analgesic agents can be used in the subject invention.

### Phenol and Menthol

Phenol and/or menthol can be administered at the surgical incision, open wound, or injection site to be treated. Phenol and menthol can be administered prior to administration of the capsaicinoid, or can be co-administered with the capsaicinoid. The effective 24 hour intracapsular capsular concentration for bolus injected phenol and menthol concentrations should be ∼25 µg/ml or ∼250 µg for a joint fluid injection volume of 10 ml.

| **Ingredients** | **MW** | **Oil Soluble** | **Aqueous Soluble** | **Log O/W** | **MP (°C)** | **Onset of Action** |
|---|---|---|---|---|---|---|
| Capsaicin | 305.41 | soluble | insoluble | 3.327 | 62-67 | moderate |
| Ethyl Alcohol | 46.07 | soluble | miscible | -0.18 | -114 | fast |
| Phenol | 94.11 | soluble | soluble | 10 | 46 | fast |
| Menthol | 156.26 | soluble | slightly soluble | 2.66 | 42 | fast |
| Lidocaine | 234.34 | soluble | insoluble | 2.359 | 68 | slow |
| Prilocaine | 220.31 | soluble | sparingly | 2.11 | 137 | slow |
| Benzocaine | 165.19 | soluble | sparingly | 1.95 | 90 | moderate |

Both phenol and menthol (1) rapidly *penetrate* the surfaces in contact with the injected formulation; and (2) serve to *reduce or eliminate the acute burning and stinging pain sensation* associated with the administration of the TRPV1 agonist (e.g. capsaicin). Eugenol can also be used. The subject invention may include the use of specific injectable analgesics (e.g. phenol and menthol) that have a fast "onset of action" relative to capsaicin to effectively moderate the burning sensation effect of capsaicin. Onset of action of a compound is linked to its physicochemical properties; some agents are listed in Table III.

### TABLE III - Onset of Action of Selected Analgesic and Anesthetic Ingredients

The use of these selected analgesics with a fast onset of action effectively moderates the burning effect of capsaicin when concomitantly administered, but also provides more immediate pain relief relative to capsaicin. In one embodiment of the invention, the injected analgesic agent has a molecular weight of 160 or less. Capsaicin provides more long term / long lasting pain relief relative to these fast onset of action injectable analgesics.

Local anesthetics such as lidocaine, are common additions to intra articular injections. They are used in many basic medical procedures to produce numbness for a short period of time, including being used in doctor's offices or at hospitals to numb an area that is injured or that requires minor surgical manipulation. A local anesthetic agent can be added to the injectable vehicle to provide localized pain relief. Examples of anesthetics are lidocaine, bupivacaine, ropivacaine, dibucaine, procaine, chloroprocaine, prilocaine, mepivacaine, etidocaine, tetracaine, and xylocale. Advantageously, the anesthetic is administered prior, i.e. 2-20 minutes, to the capsaicinoid administration due to differences in the onset of action of the compounds.

### Anti-inflammatory Agents

An anti-inflammatory agent is optionally included in the formulation.

### Non-Steroidal Anti-Inflammatory Drugs (NSAIDs)

Non-steroidal anti-inflammatories, such as aspirin, naproxin, indomethacin, diclofenac sodium, refecoxib, and ibuprofen, inhibit the enzyme cyclooxygenase (COX-2 inhibitor) and therefore decrease prostaglandin synthesis. Prostaglandins are inflammatory mediators that are released during allergic and inflammatory processes. In whole, the NSAIDs prevent the prostaglandins from ever being synthesized, reducing or eliminating the pain.

An NSAID anti-inflammatory agent, such as diclofenac, aspirin, ibuprofen, naproxen, benoxaprofen, flurbiprofen, fenoprofen, and others, can be added to the injectable composition to provide localized pain relief with minimal or no systemic absorption.

### Corticosteroids

In one example of a medical use of the compositions of the invention, an adjunctive agent such as a corticosteroid (glucocorticoid) is administered prior to, or concurrently with, capsaicin to attenuate an initial acute burning and stinging pain from the administered dose of capsaicin. Some of the most potent anti-inflammatories are the corticosteroids. Corticosteroids have been shown to reduce inflammation by inhibiting the production of substances that cause inflammation. The use of injectable corticosteroids is widespread in pain management. These drugs can diminish or eliminate painful foci by virtue of their anti-inflammatory properties. Corticosteroid injection can be highly effective because it delivers the medication directly to the site of inflammation. The corticosteroid injection is an effective way to alleviate inflammation including that resulting from the inflammatory substances from the TRPV-1 nerve endings due to the presence of capsaicin. While not wishing to be bound by theory, it is believed that administration of the corticosteroids works by calming nerves and reducing the inflammatory effects of certain bio-transmitters, such as substance P and bradykinin, resulting from the capsaicinoid administration.

There are several corticosteroids that can be used including dexamethasone, methylprednisolone acetate, methylprednisolone sodium succinate and mixtures thereof. Other equivalent corticosteroids known to those skilled in the art can also be used. In one embodiment, the corticosteroid solubility is increased by the formation of a corticosteroid concentrate (e.g. using PS 80 or polyoxy 40 hydrogenated castor oil) according to US Patent 8637,569.

Injectable corticosteroids are available in either water-soluble or depot formulations. Water-soluble corticosteroids are typically not used for intra-articular injections because they rapidly diffuse from the injected area, exerting systemic effects. Depot formulations remain for a longer period of time at the injected site, maintaining a local effect, and are advantageous for joint injections. There are a variety of depot corticosteroids available for use including methyl prednisolone acetate, betamethasone sodium phosphate, betamethasone acetate, hydrocortisone acetate, prednisolone tebulate, triamcinolone acetonide, triamcinolone hexacetonide and mixtures thereof. Corticosteroids with lower solubility compared to compounds with greater solubility have an added benefit of maintaining effective synovial levels for a longer time and produce lower systemic levels.

When a local corticosteroid is administered with the capsaicinoid, the administration provides burning and stinging pain relief to the area to be treated. The administration of the corticosteroid with the administration of capsaicin or capsaicin-like compounds results in less pain upon capsaicin administration, and pain relief at the site for a prolonged period of time - greater than 1 month, advantageously greater than 3 months, and most advantageously, greater than 6 months.

The use in orthopedic surgery of corticosteroids with their potent anti-inflammatory activity, with capsaicinoids, eliminates or substantially reduces the acute pain from capsaicinoid administration. This allows extended pain relief in a safe and effective manner.

### Aqueous Based Capsaicin Injectable Formulations - Micellar/Free

The formulations cited in Table IV rely on the PEG and ethyl alcohol to solubilize capsaicin and the analgesic agents, phenol and menthol. A nominal content of ∼ 15 - 40 wt. % PEG and ∼ 0 - 40 wt. % ethyl alcohol are required to maintain a single-phase aqueous solution of phenol and menthol.

**TABLE IV - High PEG 300/400 Capsaicin Formulations (micellar free)**

| **INGREDIENT** | **FUNCTION** | **CONCENTRATION RANGE - (wt.%)** | **Preferred Conc. (wt. %)** |
|---|---|---|---|
| **Capsaicin** | **Defunctionalization of TRPV-1 sensory neurons** | **0.0002 - 0.1** | **∼0.01** |
| **PEG 300/400** | **Solubilizing agent** | **15 - 20** | **∼30** |
| **Hyaluronic Acid** | **Viscosity enhancer, stabilizing & moisturizing agent** | **0.25 -1.5 (can vary the range of Molecular weights to achieve desired viscosity preferably 800 -1,500 kDaltons)** | **∼1.0 (1,000 kDaltons)** |
| **Phenol, USP** | **Analgesic/anesthetic and antiseptic agent** | **0 - 1** | **∼0.1** |
| **Eugenol, USP** | **Analgesic/anesthetic and antiseptic agent** | **0 - 1** | **∼0.1** |
| **Menthol, USP** | **TRPV-8 Cooling & analgesic agent** | **0 - 1** | **∼0.1** |
| **Sodium Chloride/ Sucrose** | **Tonicity agents** | **NaCl - < 0.9** | **NaCl - ∼ 0.9** |
| | | **Sucrose - ∼ 5** | **Sucrose - 5** |
| **Water** | **Solvent (pyrogen free)** | **q.s.** | **q.s.** |
| **Phosphate Buffer / Citrate Buffer** | **pH Control** | **Adjust pH from 7.0 -8.0** | **∼7.2** |
| **Diclofenac Sodium (a NSAID) - optional** | **Anti-inflammatory agent** | **0 - 1** | **∼ < 1** |

### Aqueous Based Capsaicin Injectable Formulations - Containing Capsaicin in Micelles

As noted in Examples V and Examples VI A to VI D of US patent 8,637,569, compositions containing 1mg/ml of capsaicin within an aqueous solution required 10 mg/ml of PS80 in aqueous solutions. It was also noted that for each 10 mg/ml increase in the PS 80 concentration that the aqueous solubility of capsaicin increases by about 1 mg/ml.

The examples of commonly owned U.S. Patent 8,637,569 however, unexpectedly teach requiring one-half (½) the PS 80 concentration within an aqueous solution; i.e., 5 mg/ml of PS 80 to solubilize 1mg/ml of capsaicin. The proportional 1 mg/ml concentration in the relatively aqueous insoluble capsaicin achieved with 5 mg/ml of PS 80 indicates that capsaicin is contained within micelles. (See Example 1) Therefore, to achieve a capsaicin concentration of 0.06 mg/ml, only 0.3 gm/ml of PS 80 is required.

The formulations cited in Table V below rely on the PS 80 to dissolve capsaicin via solubilizing and micellar formation and the PEG and ethyl alcohol content to solubilize the analgesic agents, phenol and menthol. A 5 - 20 wt. % of PEG and 0 - 40 wt. % ethyl alcohol are required to maintain a single-phase aqueous solution of phenol and menthol.

**TABLE V - PS 80 Capsaicin Formulations**

| **INGREDIENT** | **FUNCTION** | **CONCENTRATION RANGE - (wt.%)** | **Preferred Conc. (wt. %)** |
|---|---|---|---|
| **Capsaicin** | **Defunctionalization of TRPV-1 sensory neurons** | **0.0002 - 0.1** | **∼0.01** |
| **Polysorbate 80** | **Capsaicin Solubilizing via micelles** | **0.001-10** | **∼0.5** |
| **PEG 300/400** | **Solubilizing agent** | **5 - 20** | **∼15** |
| **Hyaluronic Acid** | **Viscosity enhancer, stabilizing & moisturizing agent** | **0.25 -1.5 (can vary the range of Molecular weights to achieve desired viscosity preferably 800 -1,500 kDaltons)** | **∼1.0 (1,000 kDaltons)** |
| **Phenol, USP** | **Analgesic/anesthetic and antiseptic agent** | **0 - 1** | **∼0.1** |
| **Eugenol, USP** | **Analgesic/anesthetic and antiseptic agent** | **0 - 1** | **∼0.1** |
| **Menthol, USP** | **TRPV-8 Cooling & analgesic agent** | **0 - 1** | **∼0.1** |
| **Sodium Chloride/ Sucrose** | **Tonicity agents** | **NaCl - < 0.9** | **NaCl - ∼ 0.9** |
| | | **Sucrose - ∼ 5** | **Sucrose - 5** |
| **Water** | **Solvent (pyrogen free)** | **q.s.** | **q.s.** |
| **Phosphate Buffer / Citrate Buffer** | **pH Control** | **Adjust pH from 7.2 -8.0** | **∼7.2** |
| **Diclofenac Sodium (a NSAID) - optional** | **Anti-inflammatory agent** | **0 - 1** | **- < 1** |

The formulations cited in Table VI below rely on Cremophor® RH 40 (alternatively, Cremophor® ELP, or Solute® HS 15) to solubilize capsaicin and the analgesic agents, phenol and menthol.

**TABLE VI - Cremophor® RH 40 Capsaicin Formulations**

| **INGREDIENT** | **FUNCTION** | **CONCENTRATION RANGE - (wt.%)** | **PREFERRED CONC. (wt.%)** |
|---|---|---|---|
| **Capsaicin** | **Defunctionalization of TRPV-1 sensory neurons** | **0.0002 - 0.1** | **∼0.01** |
| **Cremophor® RH 40 or Cremophor® ELP, or Solute® HS 15** | **Solubilizing Complexing Agents via micelle formation** | **0.001 - 1.0** | **∼0.06** |
| **Hyaluronic Acid** | **Viscosity enhancer, Stabilizing and moisturizing agent** | **0.25-1 (can vary the range of Molecular weights to achieve desired viscosity preferabley 800-1,500 kDaltons)** | **∼0.5 (∼ 1,000 kDaltons)** |
| **Phenol, USP** | **Analgesic/anesthetic and antiseptic agent** | **0 - 1** | **∼0.1** |
| **Menthol, USP** | **Analgesic/anesthetic and antiseptic agent** | **0-1** | **∼0.1** |
| **Eugenol, USP** | **Analgesic/anesthetic and antiseptic agent** | **0-1** | **∼0.1** |
| **Sodium Chloride / Sucrose** | **Tonicity agents** | **NaCl - < 0.9** | **NaCl - ∼ 0.9** |
| | | **Sucrose - ∼ 5** | **Sucrose - ∼ 5** |
| **Water** | **Solvent (pyrogen free)** | **q.s.** | **q.s.** |
| **Phosphate Buffer/Citrate Buffer** | **pH Control** | **Adjust pH from 7.2 - 8.0** | **∼7.2** |
| **Diclofenac Sodium (a NSAID) - optional** | **Anti-inflammatory agent** | **0 - 1** | **∼ < 1** |

### Methods of Making the Formulations of the Invention

Methods of making the formulations of the invention are discussed in Examples 3 and 6 below.

### Methods of Using the Compositions of the Invention and Administration

Aqueous capsaicinoid formulations according to the invention may be administered for treatment of pain. A single dose of the capsaicinoid formulation may be administered at a discrete site, a surgical site or open wound in an amount effective to denervate the injection site, surgical site (e.g. laparoscopy) or wound (e.g. bone fracture or torn ligament). Examples of sites are joints, muscles, tendons, nerves or tumors.

The formulations of the present invention are useful in 1) relieving pain at an intra-articular site or at a body space; 2) alleviating the post surgical pain experienced by patients following discharge from a clinical care facility; 3) providing effective post-surgical analgesia such that the amount of narcotics taken by a patient is reduced thereby decreasing post-surgical rehabilitation time.

Uses and methods and of the compositions of the invention include but are not limited to orthopedic disorders of the knee, shoulder, hip, back, spine, neck, elbows, hand, foot and other disorders which involve pain at a specific site. Examples of intra-articular administration include injection to the knee, elbow, hip, carpal, tarsal, wrist, intervertebral disk, ankle, and any other joints subject to arthritic conditions. Examples of body spaces include bursae or peritoneum.
Osteoarthritis (OA) is associated with a loss of cartilage. Cartilage is composed of specialized cells called chondrocytes that produce a large amount of extracellular matrix composed of collagen fibers, abundant ground substance rich in proteoglycan, and elastin fibers.

The compositions of the invention can be used for management and prolonged relief of nociceptive pain in mammals associated with osteoarthritis, rheumatoid arthritis, tendonitis, bursitis, pre- and post-surgical conditions, as well as for neuropathic pain occurring secondary to nerve injury. Other uses and methods of administration are described in US patent 8,420,600, and commonly owned US Ser. No. 13/609,100.

As used herein, "injection" means administration to a site through the skin. "Implantation" shall mean administration at a site by embedding a dose of material into the skin, muscle, tendon or joint.

The capsaicin formulations can be administered via injection or infiltration to a site. For surgery or wounds, the dose is administered to the muscle, tissue or bones surrounding the surgical or wound site. When the capsaicinoid is administered by infiltration, the capsaicinoid is administered to the surgical site or wound with an instrument known to those skilled in the art for administering via infiltration, e.g. a needle and syringe.

As used herein, "therapeutically effective amount" refers to that quantity or dose of an agent to produce a clinically desired result such as a biological response, or a reduction of a symptom of a disease or condition, e.g. reduction in or elimination of pain.

When the single dose of capsaicin is administered via injection, the injection volume of capsaicin depends on the localized site of administration. Suitable injection volumes to be delivered advantageously range from about 0.1 to about 20 ml, more advantageously from about 0.5 to about 10 ml and most advantageously from about 1.0 to about 5 ml, depending on the site to be treated.

Advantageous use of the injectable compositions of the invention for treatment of joint pain and to minimize or eliminate the burning and stinging effects of injectable capsaicin follows. Sterile technique must be used for injections in order to reduce the risk of infection. The skin is initially cleaned with Betadine or other suitable antiseptic agent.

### Injection Procedure

The following procedures can be used for injection of capsaicin formulation to a joint (e.g. knee):
1. A needle is inserted into the joint and excess fluid is withdrawn.
2. An anesthetizing agent (1% Lidocaine) is injected into the affected joint and allowed to infiltrate the surrounding intra-articular surfaces for about 5 to 10 minutes. During this time period, the subject is asked to walk, flex and extend (bend) the knee to distribute the Lidocaine. Advantageously, the anesthetic agent is not removed from the joint prior to administration of the capsaicinoid formulation. A cooling means such as an ice pack or gel pack is optionally applied to the affected knee prior to, during, and/or after the Lidocaine injection.
3. Five to 10 minutes following the Lidocaine injection, the capsaicin formulation is injected at a dose of 0.2 to 0.5 mg in 5 ml solution. Ice packs or gel packs or a cooling device are optionally applied to the affected knee prior to, during and after the capsaicin formulation injection. To distribute the capsaicin in the joint, the knee is fully flexed and extended repeatedly for the first 1 minute (advantageously 30 seconds) while the subject is on the examination table. The subject is also asked to walk and flex the knee.

### Flexing of Joint after Capsaicin Injection

Significantly, movement of the joint by bending (flex and extend) the joint and/or walking within the first minute after capsaicin administration serves to circulate the formulation and reduce the burning and stinging pain.

### Cooling

Cooling the joint or administration site via external cooling means such as gel pack or ice pack or cooling device also reduces the painful effects from capsaicinoid administration. The cooling or gel pack may produce a temperature above freezing, e.g. 38-50 degrees F.

Methods of treatment may utilize capsaicin (e.g. trans capsaicin) dose ranges from 0.001 mg to 1.0 mg., advantageously from 0.2 mg. to 0.4 mg. The methods may utilize an aqueous vehicle in a volume from about 0.1 ml to 25 ml, advantageously 5-10 ml. The amounts of capsaicinoid and vehicle used for small joint or non-joint applications will vary as determined by a person skilled in the art.

The compositions of the invention include 0.0002% - 0.1% by weight of a capsaicinoid, advantageously 0.01-0.05 % by weight capsaicinoid.

The formulations of the subject invention may provide pain relief for 2 to 5 days, advantageously 7 to 21 days, or more advantageously 6 to 8 weeks, or greater than 14 weeks (i.e. greater than 1 month, advantageously greater than 3 months, and most advantageously, greater than 6 months).

Similar techniques can be used for the relief of pain relief other than joint pain.

The following examples are illustrative, but not limiting of the compositions and methods of the present invention, with the exception of examples 1-2 and 4-5, which are disclosed but do not form part of the present invention as defined by the appended claims.

### EXAMPLE 1

### Preparation of a Concentrated Capsaicin/PS 80 Solution - not according to the present invention

U.S. Patent 8,637,569 teaches 5 mg/ml of PS 80 are required to solubilize 1mg/ml of capsaicin; i.e., to achieve a capsaicin concentration of 0.06 mg/ml, only 0.3 gm/ml of PS 80 are required. Also, it was determined that the relatively aqueous insoluble capsaicin is contained within micelles since for each 1 mg/ml increase in the capsaicin solubility, 5 mg/ml of PS 80 was required. The teachings of this Patent were utilized in solubilizing capsaicin in a high purity PS 80 obtained from Croda Inc.

### STEP I - THE PREPARATION OF A CAPSACIN and PS 80 CONCENTRATE

### Ingredients Include:

- 10 grams of Polysorbate 80 (PS 80), Super refined, Croda Inc., CAS # 9005-65-6
- 2 grams of Trans-Capsaicin, Aversion Technologies Inc., USP 30, 95.7% Trans-Capsaicin, Balance Cis-Capsaicin

### Procedure:

1. 10 grams of PS 80 are added to a 50 ml "Pyrex" glass vial.
**2.** 2 grams of Trans-Capsaicin are added to the PS 80 in Step 1.
**3.** The mixture from Step 2 is heated to ∼ 125 °C to dissolve the Trans-Capsaicin and form the Trans-Capsaicin/PS80 concentrate.

The addition of a few capsaicin particulates to the room temperature Capsaicin/PS 80 solution did not result in capsaicin precipitates being formed thus indicating that the capsaicin solution was not supersaturated. Unexpectedly high concentrations levels were achieved; i.e., 200 mg capsaicin in 1.0 ml of PS 80 (200 mg/ml). The addition of 2 ml of this capsaicin/PS 80 concentrate to 98 grams of∼ 70 °C water resulted in a cloudy solution which gradually became crystal clear as the solution mixture cooled to room temperature. Thus the addition of this 20% capsaicin/PS 80 concentrate to water resulted in an aqueous capsaicin solubility level of 4 mg/ml. Similarly, the addition of 10 ml of this capsaicin/PS80 concentrate to 90 grams of∼ 70 °C water resulted in a cloudy solution which gradually became crystal clear as the solution mixture cooled to room temperature. Thus, the addition of this 2% capsaicin/PS 80 concentrate to water resulted in an aqueous capsaicin solubility level of 20 mg/ml.

It was also noted that for each 5 mg/ml increase in the PS 80 concentration that the aqueous solubility of capsaicin increases by about 1 mg/ml. While not wishing to be bound by theory, this suggests that the capsaicin is contained in PS 80 micelles.

### EXAMPLE 2 - not according to the present invention

### Preparation of a 200 gram Aqueous Solution Containing the Capsaicin/PS80 Concentrate from Example 1 and Hyaluronic Acid

### STEP I - THE PREPARATION OF THE HYALURONIC ACID SOLUTION

### Ingredients Include:

- 1 gram of Hyaluronic Acid, M.W. = 1,000 kDaltons, Lotioncrafter LLC, CAS # 9067-32-7
- 191.8 gram of Distilled Water, Poland Springs

### Procedure:

**1.** Add 1 gram of Hyaluronic Acid in a 400 cc Pyrex beaker.
**2.** Add 191.8 grams of water to the Hyaluronic Acid and thoroughly mix until a clear solution is obtained. It takes an extended time duration to completely solubilized the hyaluronic acid.

### STEP II - THE PREPARATION OF THE 0.6 mg/ml Capsaicin, 3.0 mg/ml PS80 & 5 mg/ml Hyaluronic Acid Aqueous Solution

### Ingredients Include:

- 7.2 grams of the Capsaicin/PS 80 concentrate from Example 1.
- 192.8 grams of the Hyaluronic Acid from STEP I.

### Procedure:

**1.** To the 192.8 grams of the Hyaluronic Acid Solution prepared in STEP I and contained within a 400 cc Pyrex beaker, slowly add 7.2 grams of the Capsaicin/PS 80 concentrate prepared in Example 1 while thoroughly stirring.
**2.** The mixture from Step 1 is now ready for subsequent packaging.

After thoroughly mixing the ingredients, the resulting mixture was crystal clear and was moderately viscous. The viscosity of the mixture can be adjusted by varying the hyaluronic acid content.

The elevated temperature solubilization of capsaicin in PS 80 is important. Several attempts to add the PS 80 and capsaicin to water followed by heating to temperatures of ∼ 70 °C - 90 °C resulted in only a fraction of the capsaicin dissolving.

The ability to achieve increased capsaicin solubility levels within an aqueous medium as described in the foregoing text, allows for significant reductions in the liquid volume of capsaicin to be injected. Experiments with PEG-400 did not achieve the capsaicin solubility levels when compared to the PS80 (micellar solubilization) surfactant.

### EXAMPLE 3

### Preparation of 200 grams of a 0.06 mg/ml Capsaicin Injectable Solution (micelle free)

### STEP I - THE PREPARATION OF A CAPSACIN, PHENOL, MENTHOL SOLUTION

### Ingredients Include:

- 60 grams of PEG-300, Spectrum Chemical, NF, CAS # 25322-68-3
- 16 grams of Ethyl Alcohol, Graves Grain Alcohol, 190 Proof
- 0.2 grams of Phenol, Liquefied (carbolic Acid), Spectrum Chemical, USP, CAS # 108-95-2
- 0.2 grams of L-Menthol, Crystal, Spectrum Chemical, USP, CAS # 2216-51-5
- 0.012 grams of Trans-Capsaicin, Aversion Technologies Inc., USP 30, 95.7% Trans-Capsaicin, Balance Cis-Capsaicin

### Procedure:

**1.** Add 60 grams of PEG-300 in a 400 cc Pyrex beaker.
**2.** Add 16 grams of Ethyl Alcohol to the PEG-300 & thoroughly mix.
**3.** Add 0.2 grams of Liquefied Phenol to the mixture of Step 2.
**4.** Add 0.2 grams of L-Menthol Crystals to the mixture of Step 3.
**5.** Add 0.012 grams of Trans-Capsaicin to the mixture of Step 4
**6.** Heat the mixture of Step 5 to ∼ 40 °C to hasten the formation of the solution of menthol and capsaicin.
**7.** The solution from Step 6 is set aside and allowed to cool to room temperature.

### STEP II - THE PREPARATION OF THE HYALURONIC ACID SOLUTION

### Ingredients Include:

- 1 gram of Hyaluronic Acid, M.W. = 1,000 kDaltons, Lotioncrafter LLC, CAS # 9067-32-7
- 122.6 gram of Distilled Water, Poland Springs

### Procedure:

**1.** Add 1 gram of Hyaluronic acid in a 250 cc Pyrex beaker.
**2.** Add 122.6 grams of water to the Hyaluronic Acid and thoroughly mix until a clear solution is obtained. It takes an extended time duration to completely solubilized the hyaluronic acid; mostly likely 30 minutes.

### STEP III - THE COMBINING OF STEP I & STEP II SOLUTIONS

### Ingredients Include:

- The solution mixture from STEP I
- The solution mixture from STEP II.

### Procedure:

**1.** The hyaluronic acid solution from STEP II is slowly added to the capsaicin containing solution from Step I while thoroughly stirring.
**2.** The mixture from Step 1 is now ready for subsequent packaging.

Table 6 contains the ingredients contained within the 0.06 mg/ml capsaicin solution.

### EXAMPLE 4 - not according to the present invention Preparation of 200 grams of a 0.06 mg/ml Capsaicin/PS 80 Micelle Injectable Solution

### STEP I - THE PREPARATION OF A CAPSACIN and PS 80 SOLUTION

### Ingredients Include: (Note: To ensure weighing accuracy, the amount of the Capsaicin/PS 80 solution prepared was 8 times that required for a 0.06 mg/ml injectable solution)

- 2 grams of Polysorbate 80 (PS 80), Super refined, Croda Inc., CAS # 9005-65-6
- 0.125 grams of Trans-Capsaicin, Aversion Technologies Inc., USP 30, 95.7% Trans-Capsaicin, Balance Cis-Capsaicin

### Procedure:

**1.** 2 grams of PS 80 are added to a 16 ml Pyrex glass vial.
**2.** 0.125 grams of Trans-Capsaicin are added to the PS 80 in Step 1.
**3.** The mixture from Step 2 is heated to ∼ 55 °C to dissolve the Trans-Capsaicin and form the Trans-Capsaicin/PS 80 solution.

### STEP II - THE PREPARATION OF THE CAPSAICIN/PS 80, PEG-300, ETHYL ALCOHOL, PHENOL & MENTHOL SOLUTION

### Ingredients Include:

- 20 mg of PEG PEG-300, Spectrum Chemical, NF, CAS # 25322-68-3
- 10 grams of Ethyl Alcohol, Graves Grain Alcohol, 190 Proof
- 0.27 grams of the Trans-Capsaicin/PS 80 solution from STEP I.
- 0.2 grams of Phenol, Liquefied (carbolic acid), Spectrum Chemical, USP, CAS # 108-95-2
- 0.2 grams of L-Menthol, Crystal, Spectrum Chemical, USP, CAS # 2216-51-5

### Procedure:

**1.** Add 20 grams of PEG-300 in a 400 cc Pyrex beaker.
**2.** Add 10 grams of Ethyl Alcohol to the PEG-300 and thoroughly mix.
**3.** Add 0.27 grams of the Trans-Capsaicin/PS 80 solution to the mixture from Step 2 and thoroughly stir.
**4.** Add 0.2 grams of Liquefied Phenol to the mixture of Step 3.
**5.** Add 0.2 grams of L-Menthol Crystals to the mixture of Step 4.
**6.** Heat the mixture from Step 5 to ∼ 40 °C to hasten the solution of menthol.
**7.** The solution from Step 5 is set aside and allowed to cool to room temperature

### STEP III - THE PREPARATION OF THE HYALURONIC ACID SOLUTION

### Ingredients Include:

- 1 gram of Hyaluronic Acid, M.W. = 1,000 kDaltons, Lotioncrafter LLC, CAS # 9067-32-7
- 169.5 grams of Distilled Water, Poland Springs

### Procedure:

**1.** Add 1 gram of Hyaluronic acid in a 400 cc Pyrex beaker.
**2.** Add 169.5 grams of water to the Hyaluronic Acid and thoroughly mix until a clear solution is obtained. It takes an extended time duration to completely solubilized the hyaluronic acid.

### STEP III - THE COMBINING OF STEP I & STEP II SOLUTIONS

### Ingredients Include:

- The solution mixture from STEP I
- The solution mixture from STEP II.

### Procedure:

**1.** The hyaluronic acid solution from STEP II is slowly added to the capsaicin containing solution from STEP II while thoroughly stirring.
**2.** The mixture from Step 1 is now ready for subsequent packaging.

Table 6 contains the ingredients contained within the 0.06 mg/ml capsaicin solution.

### EXAMPLE 5 - not according to the present invention

### Preparation of 200 grams of a 4mg/ml Capsaicin, 1mg/ml Menthol and 1mg/ml Phenol Transparent Aqueous Concentrate

The following steps were followed in the preparation of a 4 mg/ml Capsaicin, 1 mg/ml Menthol and 1 mg/ml Phenol concentrate.

### Ingredients Include:

- 0.40 grams of the Trans-Capsaicin, Aversion Technologies Inc., USP 30, 95.7% Trans-Capsaicin, Balance Cis-Capsaicin
- 0.10 grams of Phenol, Liquefied (carbolic acid), Spectrum Chemical, USP, CAS # 108-95-2
- 0.10 grams of L-Menthol, Crystal, Spectrum Chemical, USP, CAS # 2216-51
- 2 grams Cremophor® RH 40, CAS Number 61788-85-0 obtained from Sigma Aldrich, St. Louis, MO

### Procedure:

**1.** Add 0.4 grams of capsaicin powder, 0.1 grams of menthol crystals and 0.1 grams of liquefied phenol to a 300 cc Pyrex beaker.
**2.** Add 2.0 grams of Cremophor® RH 40 to the mixture from Step 1.
**3.** The mixture from Step 2 is heated to about 125 °C.
**4.** Water is slowly added to the mixture from Step 3 while thoroughly stirring to provide an aqueous mixture weighing 200 grams.

An optically clear aqueous solution containing 4 mg/ml Capsaicin, 1 mg/ml Menthol, 1 mg/ml Phenol and 20 mg/ml Cremophor® RH 40 resulted. A combined weight ratio of the capsaicin, menthol and phenol mixture to the Cremophor® 40 surfactant was 1.0/3.33.

### EXAMPLE 6

### Preparation of the HA/PEG/Capsaicin Injection Solution

The capsaicin used in the injectable formulation is a high purity synthetic Trans-Capsaicin product intended for drug use. Formosa Laboratories is the certified manufacturer (DMF #16769). Johnson Compounding of Waltham, MA prepared the capsaicin injection formulation as a sterile liquid and Bioventus LLC of Durham, NC supplied the sterile hyaluronic acid (HA), Supartz, formulation used for preparing the sterile capsaicin-HA injection solution. The capsaicin solution and Supartz were manufactured and released in compliance with Good Manufacturing Practices (GMPs).

The capsaicin solution is manufactured by dissolving capsaicin in 100% polyethylene glycol 300 (PEG). The resulting solution was sterile filtered and aseptically filled into 5 ml Type I glass vials that that were subsequently sealed. The components and compositions of the capsaicin solution are listed in Table VII.

**Table VII - Components of the Capsaicin Solution**

| **Ingredients** | **Concentration** | **Function** |
|---|---|---|
| 99⁺ wt% trans-capsaicin¹ | 0.04 wt% (0.4 mg/ml) | API |
| PEG-300 P h Eur² | q.s. ad | Solvent |

| | | |
|---|---|---|
| ¹The cGMP 99⁺ wt% trans-capsaicin was procured from Formosa Laboratories, Taiwan ²Sigma-Aldrich, St. Louis, MO (Catalog #81162) | | |

The capsaicin solution was then mixed with Supartz. Supartz is a sterile, viscoelastic non-pyrogenic solution of purified, high molecular weight (620,000-1,170,000 Daltons) sodium hyaluronate (hyaluronan) having a pH of 6.8-7.8. Each one ml of Supartz contains 10 mg of sodium hyaluronate (hyaluronan) dissolved in a physiological saline (1.0% solution). The sodium hyaluronate (hyaluronan) is extracted from chicken combs. Sodium hyaluronate (hyaluronan) is a polysaccharide containing repeating disaccharide units of glucuronic acid and N-acetylglucosamine. The components and composition of Supartz are shown in Table VIII.

**Table VIII - Components of Supartz**

| **Ingredients** | **Concentration (wt %)** | **Function** |
|---|---|---|
| Sodium Hyaluronate (hyaluronan) | 1.0 | Solvent |
| Sodium Chloride | 0.85 | Isotonic Agent |
| Dibasic Sodium Phosphate Dodecahydrate | 0.05 | Buffering Agent |
| Sodium Dihydrogen Phosphate Dihydrate | 0.002 | Buffering Agent |
| Water | q.s. ad | Solvent |

Capsaicin concentration of the aqueous capsaicin-HA sterile injection solution ranged from 0.2-0.5 mg of capsaicin in 5 ml sterile injection solution and was prepared by mixing the capsaicin solution with the hyaluronic acid formulation, Supartz, just prior to injection into the intra-articular knee joint.

### EXAMPLE 7

### Injection of the HA/PEG/Capsaicin Solution into the Knee

A 72 year old male with painful osteoarthritis of the knees received an injection of an 8 ml solution containing 1% Lidocaine into the intra-articular cavity of his left knee. The Lidocaine solution was distributed throughout the joint cavity by walking and bending of the knee for about 5 minutes prior to intra-articular injection of 5 ml of the aqueous capsaicin-HA sterile solution. A capsaicin dose level of 0.2 mg capsaicin was achieved by mixing 0.5 ml of the capsaicin solution with 4.5 ml of the HA containing Supartz vehicle. The knee had been pre-cooled for about 3 minutes prior to the injection of the aqueous capsaicin-HA sterile solution and cooling of the knee was continued for the remainder of the treatment procedure. Immediately after the capsaicin injection, the knee was bent (flexed and extended) vigorously for about 1 minute to distribute the aqueous capsaicin-HA sterile solution within the joint cavity. The pain level briefly rose to a moderate level quickly subsided upon resting and bending the knee. Seven minutes after injection of the aqueous capsaicin-HA sterile solution, the procedures were completed and the subject experienced complete relief of his osteoarthritic pain symptoms. Table IX summaries of procedural pain levels as a function of elapsed time.

**Table IX - INJECTED KNEE # 1**

| **0.5 ml Capsaicin solution and 4.5 ml Supartz** **(0.2 mg Capsaicin dose level)** | | |
|---|---|---|
| **ELAPSED TIME (Minutes)** | **PROCEDURE** | **PAIN SCORE (0 - 10)** |
| **0** | **Cold pack applied to knee & Inject 8cc 1% Lidocaine, Walk around, flex & extend knee** | **0** |
| **7** | **Inject 5 cc Cap/Supartz mixture, flex & extend knee vigorously** | **0** |
| **8** | | **2** |
| **9** | **Vigorously flex & extend knee forabout 1 minute** | **3** |
| **10** | **Flex & extend knee** | **2** |
| **11** | | **3** |
| **12** | **Flex & extend knee** | **2** |
| **13** | | **1.5** |
| **14** | **Walking, no pain** | **0** |

| | | |
|---|---|---|
| **Note: *Elapsed time from Capsaicin Inject to complete join pain relief =* 7 *minutes*** | | |

### EXAMPLE 8

### Injection of the HA/PEG/Capsaicin Solution into the Knee

A 74 year old male with painful osteoarthritis of the knees had 14 cc of fluid removed from his left knee joint prior to injection of an 10 ml solution containing 1% Lidocaine into the intra-articular cavity of his left knee. The Lidocaine solution was distributed throughout the joint cavity by walking and bending of the knee for about 7 minutes prior to intra-articular injection of 5 ml of the aqueous capsaicin-HA sterile solution. A capsaicin dose level of 0.3 mg capsaicin was achieved by mixing 0.75 ml of the capsaicin solution with 4.25 ml of the HA containing Supartz vehicle. The knee had been pre-cooled for about 2 minutes prior to the injection of the capsaicin-HA sterile solution and cooling of the knee was continued for the remainder of the treatment procedure. Immediately after the capsaicin injection, the knee was bent vigorously for about 1 minute to distribute the aqueous capsaicin-HA sterile solution within the joint cavity. The pain level briefly rose to a relatively high level and quickly subsided upon the application of additional icing and bending of the knee. Fourteen minutes after injection of the aqueous capsaicin-HA solution, the procedures were completed and the subject experienced complete relief of his osteoarthritic pain symptoms. Table X summarizes of procedural pain levels as a function of elapsed time.

**Table X- INJECTED KNEE # 2**

| **0.75 ml Capsaicin solutin & 4.25 ml Supartz** **0.4 mg Capsaicin dose level** | | |
|---|---|---|
| **ELAPSED TIME (Minutes)** | **PROCEDURE** | **PAIN SCORE (0-10)** |
| **0** | **Remove 14 cc fluid from the joint & Inject 10cc of 1% Lidocaine, Walk around** | **0** |
| **7** | **Inject 5 cc Cap/Supartz mixture, flex & extend knee vigorously** | **6** |
| **8** | **--** | **8** |
| **10** | **--** | **5.5** |
| **11** | **--** | **4.5** |
| **12** | **Additional ice applied** | **3** |
| **13** | **--** | **3.5** |
| **14** | **Flex & extend knee** | **3** |
| **15** | **Flex & extend knee several times** | **3** |
| **15** | **--** | **3** |
| **17** | **--** | **2.5** |
| **18** | **--** | **2** |
| **19** | **--** | **2** |
| **20** | **--** | **2** |
| **21** | **Walking** | **0** |

| | | |
|---|---|---|
| **Note: *Elapsed time from Capsaicin Inject to complete join pain relief* = *14 minutes*** | | |

### EXAMPLE 9

### Injection of the HA/PEG/Capsaicin Solution into the Knee

The same 74 year old male as in Example 8 with painful osteoarthritis of the knees had 5 cc of fluid removed from his right knee joint prior to injection of an 10 ml solution containing 1% Lidocaine into the intra-articular cavity of his left knee. The Lidocaine solution was distributed throughout the joint cavity by walking and bending of the knee for about 8 minutes prior to intra-articular injection of the 5 ml aqueous capsaicin-HA sterile solution. A capsaicin dose level of 0.3 mg capsaicin was achieved by mixing 1 ml of the capsaicin solution with 4 ml of the HA containing Supartz vehicle. The knee had been pre-cooled for about 2 minutes prior to the injection of the capsaicin-HA sterile solution and cooling of the knee was continued for the remainder of the treatment procedure. Immediately after the capsaicin injection, the knee was bent vigorously for about 1 minute to distribute the aqueous capsaicin-HA sterile solution within the joint cavity. The pain level briefly rose to a relatively moderate level quickly subsided upon the and bending and resting of the knee. Eleven minutes after injection of the aqueous capsaicin-HA solution, the procedures were completed and the subject experienced complete relief of his osteoarthritic pain symptoms. Table XI summarizes of procedural pain levels as a function of elapsed time.

**Table XI - INJECTED KNEE # 3**

| **1 ml Capsaicin solution & 4 ml Supartz** **0.4 mg Capsaicin dose level el** | | |
|---|---|---|
| **ELAPSED TIME (Minutes)** | **PROCEDURE** | **PAIN SCORE (0-10)** |
| **0** | **Remove 5 cc fluid from the joint & Inject 10cc of 1% Lidocaine, Walk around** | **0** |
| **8** | **Inject 5 cc Cap/Supartz mixture, flex & extend knee vigorously** | **4** |
| **9** | **--** | **4.5** |
| **10** | **--** | **5.5** |
| **11** | **--** | **5** |
| **12** | **--** | **3.5** |
| **13** | **--** | **3.5** |
| **14** | **--** | **3** |
| **15** | **--** | **2.5** |
| **16** | **Flex & extend knee several times** | **2.5** |
| **17** | **--** | **2** |
| **18** | **Walking** | **2** |
| **19** | **--** | **0** |

| | | |
|---|---|---|
| **Note: *Elapsed time from Capsaicin Inject to complete join pain relief* = *11 minutes*** | | |

It is intended that the invention not be limited to the particular embodiment disclosed as the best mode contemplated for carrying out this invention, but that the invention will include all embodiments falling within the scope of the appended claims.

## Claims

1. An aqueous composition for injection in a mammal comprising:
0.0002 % - 0.1% by weight of a capsaicinoid,
99.9 % - 99.9998% by weight of an aqueous vehicle comprising a solubilizing agent to solubilize said capsaicinoid, and hyaluronic acid as an extended release agent to slow the release of said capsaicinoid from said aqueous composition upon injection of said composition in a mammal,
wherein said aqueous vehicle reduces or eliminates the burning and stinging created by said capsaicinoid upon injection; and either:
(A) the aqueous composition is not the capsaicin composition produced according to the method of Example 4 of WO 2014/075084 A2, which is as follows:
(I) preparing 200 grams of a 0.06 mg/ml Capsaicin/PS 80 micelle injectable solution by:
i. adding 2 grams of polysorbate 80 (PS 80, Super refined, Croda Inc., CAS # 9005-65-6) to a 16 ml pyrex glass vial;
ii. adding 0.125 grams of Trans-Capsaicin (Aversion Technologies Inc., USP 30, 95.7% Trans- Capsaicin, Balance Cis-Capsaicin) to the polysorbate 80 from step (i)
iii. heating the mixture formed from step (ii) at about 55°C to dissolve the Trans-Capsaicin to form a Trans-Capsaicin/PS 80 concentrate;
(II) preparing a capsaicin/PS 80, PEG-300, ethyl alcohol, phenol & menthol solution by:
iv. adding 20 mg of PEG-300 (Spectrum Chemical, NF, CAS # 25322-68-3) to a 400 cc Pyrex beaker;
v. adding 10 grams of Ethyl Alcohol (Graves Grain Alcohol, 190 Proof) to the PEG-300 and mixing;
vi. adding 0.27 grams of the Trans-Capsaicin/PS 80 concentrate to the mixture formed from step (v) and stirring;
vii. adding 0.2 grams of Phenol (Liquefied (carbolic acid), Spectrum Chemical, USP, CAS # 108-95-2) to the mixture formed from step (vi);
viii. adding 0.2 grams of L-Menthol Crystal (Spectrum Chemical, USP, CAS # 2216-51 -5) to the mixture formed from step (vii);
ix. heating the mixture formed from step (viii) to about 40°C and allowing to cool to room temperature to provide the capsaicin/PS 80, PEG-300, ethyl alcohol, phenol & menthol solution;
(III) preparing a hyaluronic acid solution by:
x. adding 1 gram of Hyaluronic Acid (M.W. = 1 ,000 kDaltons, Lotioncrafter LLC, CAS # 9067-32-7) to a 400 cc Pyrex beaker;
xi. adding 169.5 grams of Distilled Water, Poland Springs to the Hyaluronic Acid from step (x) and mixing until a clear solution is obtained to provide the hyaluronic acid solution
(IV) combining the capsaicin/PS 80, PEG-300, ethyl alcohol, phenol & menthol solution of step (ix) with the hyaluronic acid solution of step (xi) by:
xii. slowing added the hyaluronic acid solution of step (xi) to the capsaicin/PS 80, PEG-300, ethyl alcohol, phenol & menthol solution of step (ix) and stirring to provide the capsaicin composition produced according to the method of Example 4 of WO 2014/075084 A2;
or
(B) said solubilizing agent is polyethylene glycol-400 and/or polypropylene glycol; or glycerol.

2. An aqueous composition as in claim 1, wherein said capsaicinoid is capsaicin or trans-capsaicin.

3. An aqueous composition as in claim 1, wherein said capsaicinoid is in the form of a concentrate with a nonionic surfactant.

4. An aqueous composition as in claim 1, wherein said solubilizing agent is polyethylene glycol-400 or polypropylene glycol.

5. An aqueous composition as in claim 1, wherein said extended release agent is 0.5% - 1.5% by weight of hyaluronic acid, or wherein said hyaluronic acid has an average molecular weight of 1000 kDaltons, or wherein said hyaluronic acid is in the form of a hydrogel.

6. An aqueous composition as in claim 1 further comprising a nonionic surfactant and preferably either wherein the capsaicinoid is capsaicin and the nonionic surfactant is polyoxy 40 hydrogenated castor oil, or wherein the capsaicinoid is capsaicin and the nonionic surfactant is polysorbate 80, and preferably wherein the weight ratio of capsaicin to polysorbate 80 is 1 to 5.

7. An aqueous composition as in claim 1 further comprising an anti-inflammatory agent, preferably a corticosteroid, or further comprising an analgesic agent, preferably one or more selected from the group consisting of phenol, menthol and eugenol, or further comprising an anesthetic agent.

8. The composition of claim 1 for use in treating pain at a site in a mammal, preferably a human, by administration, preferably by injection or by infiltration to said site in said mammal of a therapeutically effective amount of the aqueous composition.

9. The composition of claim 1 for use according to claim 8 wherein the composition is for use in combination with an anesthetic to be administered prior to the administration of said aqueous composition, said anesthetic agent preferably being for administration in a pharmaceutically acceptable vehicle in a volume from 0.1 ml to 25 ml.

10. The composition of claim 1 for use according to claim 8 and further for use with application of cooling means to said site prior to, during and/or after administration of said aqueous composition.

11. The composition of claim 1 for use according to claim 8 wherein the capsaicinoid is trans-capsaicin and the dose level ranges from 0.001 mg to 0.5 mg.

12. The composition of claim 1 for use according to claim 8 wherein said aqueous composition is for administration in a volume from 0.1 ml to 25 ml.

13. A kit for administering a capsaicinoid by intra articular injection comprising:
a) at least one unit dose of an anesthetic agent
b) at least one unit dose of an aqueous composition according to any one of claims 1 to 7,
wherein said kit is arranged such that said anesthetic agent is administered prior to said aqueous solution, preferably wherein the kit further comprises a means for administration of a) and b) or preferably wherein the kit further comprises instructions for administration or preferably wherein the capsaicinoid, hyaluronic acid and anesthetic are formulated together.

## Patentansprüche

1. Wässrige Zusammensetzung zur Injektion in ein Säugetier, umfassend:
0,0002 % - 0,1 Gew.-% eines Capsaicinoids,
99,9 % - 99,9998 Gew.-% eines wässrigen Vehikels, umfassend ein Mittel zum Solubilisieren, um das Capsaicinoids zu solubilisieren, und Hyaluronsäure als ein Mittel mit verzögerter Freisetzung, um die Freisetzung des Capsaicinoids aus der wässrigen Zusammensetzung bei Injektion der Zusammensetzung in ein Säugetier zu verlangsamen,
wobei das wässrige Vehikel das Brennen und Stechen, die durch das Capsaicinoid bei Injektion erzeugt werden, vermindert oder eliminiert; und wobei entweder
(A) die wässrige Zusammensetzung nicht die Capsaicinoid-Zusammensetzung ist, die nach dem Verfahren von Beispiel 4 der WO 2014/075084 A2 erzeugt wird, das wie folgt abläuft:
(I) Ansetzen von 200 Gramm einer injizierbaren Lösung einer 0,06 mg/ml Capsaicinoid/PS 80 Mizelle, durch
i. Zugeben von 2 Gramm Polysorbat 80 (PS 80 Super refined, Croda Inc., CAS # 9005-65-6) in eine 16 ml Pyrexglas-Fläschchen;
ii. Zugeben von 0,125 Gramm von Trans-Capsaicin (Aversion Technologies Inc., USP 30, 95,7% Trans-Capsaicin, Rest C*is*-Capsaicin) zu dem Polysorbat 80 von Schritt (i)
iii. Erhitzen der in Schritt (ii) gebildeten Mischung bei etwa 55 °C, um das Trans-Capsaicin aufzulösen, um ein *Trans*-Capsaicin/PS 80-Konzentrat zu bilden;
(II) Herstellen einer Lösung von Capsaicin/PS 80, PEG-300, Ethylalkohol, Phenol & Menthol durch:
iv. Zugeben von 20 mg PEG-300 (Spectrum Chemical, NF, CAS # 25322-68-3) in ein 400cm³ Pyrex-Becherglas;
v. Zugeben von 10 Gramm Ethylalkohol (Graves Grain Alcohol, 190-Proof) zu dem PEG-300 und Mischen;
vi. Zugeben von 0,27 Gramm des *Trans*-Capsaicin/PS 80-Konzentrats zu der in Schritt (v) gebildeten Mischung und Rühren;
vii. Zugeben von 0,2 Gramm von Phenol (verflüssigt (Carbolsäure), Spectrum Chemical, USP, CAS # 108-95-2) zu der in Schritt (vi) gebildeten Mischung;
viii. Zugeben von 0,2 Gramm von L-Menthol Crystal (Spectrum Chemical, USP, CAS # 2216-51-5) zu der in Schritt (vii) gebildeten Mischung;
ix. Erwärmen der in Schritt (viii) gebildeten Mischung bis etwa 40 °C und abkühlen lassen bis Raumtemperatur, um die Lösung von Capsaicin/PS 80, PEG-300, Ethylalkohol, Phenol & Menthol bereitzustellen;
(III) Herstellen einer Hyaluronsäure-Lösung durch:
x. Zugeben von 1 Gramm Hyaluronsäure (MG = 1000 kDalton, Lotioncrafter LLC, CAS # 9067-32-7) in ein 400 cm³ Pyrex-Becherglas;
xi. Zugeben von 169,5 Gramm destilliertem Wasser, Poland Springs, zu der Hyaluronsäure von Schritt (x) und Mischen, bis eine klare Lösung erhalten wird, um die Hyaluronsäure-Lösung bereitzustellen
(IV) Vereinen der Lösung des Capsaicin/PS 80, PEG-300, Ethylalkohols, Phenols & Menthols von Schritt (ix) mit der Hyaluronsäure-Lösung von Schritt (xi) durch:
xii. langsames Zugeben der Hyaluronsäure-Lösung von Schritt (xi) zu der Lösung von Capsaicin/PS 80, PEG-300, Ethylalkohol, Phenol & Menthol von Schritt (ix) und Rühren, um die Capsaicin-Zusammensetzung bereitzustellen, die nach dem Verfahren von Beispiel 4 der WO 2014/075084 A2 erzeugt wird;
oder
(B) das Mittel zum Solubilisieren ist Polyethylenglykol-400 und/oder Polypropylenglykol; oder Glyzerin.

2. Wässrige Zusammensetzung nach Anspruch 1, wobei das Capsaicinoid Capsaicin oder Trans-Capsaicin ist.

3. Wässrige Zusammensetzung nach Anspruch 1, wobei das Capsaicinoid in der Form eines Konzentrats mit einem nichtionischen Tensid vorliegt.

4. Wässrige Zusammensetzung nach Anspruch 1, wobei das Mittel zum Solubilisieren Polyethylenglykol-400 oder Polypropylenglykol ist.

5. Wässrige Zusammensetzung nach Anspruch 1, wobei das Mittel mit verzögerter Freisetzung 0,5% bis 1,5 Gew.-% Hyaluronsäure ist oder wobei die Hyaluronsäure ein mittleres Molekülgewicht von 1000 kDalton hat oder wobei die Hyaluronsäure in der Form eines Hydrogels vorliegt.

6. Wässrige Zusammensetzung nach Anspruch 1, ferner umfassend ein nichtionisches Tensid und bevorzugt entweder wobei das Capsaicinoid Capsaicin ist und das nichtionisches Mittel Polyoxy 40 hydriertes Rizinusöl ist, oder wobei des Capsaicinoid Capsaicin ist und das nichtionische Tensid Polysorbat 80 ist und bevorzugt wobei das Gewichtsverhältnis von Capsaicin zu Polysorbat 80 1 zu 5 beträgt.

7. Wässrige Zusammensetzung nach Anspruch 1, ferner umfassend ein entzündungshemmendes Mittel, bevorzugt ein Corticosteroid, oder ferner umfassend ein analgetisches Mittel, bevorzugt eines oder mehrere, die ausgewählt sind aus der Gruppe bestehend aus Phenol, Menthol und Eugenol, oder ferner umfassend ein anästhetisches Mittel.

8. Zusammensetzung nach Anspruch 1 zur Verwendung in der Behandlung von Schmerz an einer Stelle in einem Säugetier, bevorzugt ein Mensch, durch Verabreichung, bevorzugt durch Injektion oder durch Infiltration in Bezug auf die Stelle in dem Säugetier einer therapeutisch wirksamen Menge der wässrigen Zusammensetzung.

9. Zusammensetzung nach Anspruch 1 zur Verwendung nach Anspruch 8, wobei die Zusammensetzung zur Verwendung in Kombination mit einem Anästhetikum ist, das vor der Verabreichung der wässrigen Zusammensetzung zu verabreichen ist, wobei das anästhetische Mittel bevorzugt zur Verabreichung in einem pharmazeutisch verträglichen Vehikel in einem Volumen von 0,1 ml bis 25 ml dient.

10. Zusammensetzung nach Anspruch 1 zur Verwendung nach Anspruch 8 und ferner zur Verwendung mit Applikation von kühlenden Mitteln auf die Stelle vor, während und/oder nach der Verabreichung der wässrigen Zusammensetzung.

11. Zusammensetzung nach Anspruch 1 zur Verwendung nach Anspruch 8, wobei das Capsaicinoid *Trans*-Capsaicinoid ist und die Dosismenge im Bereich von 0,001 bis 0,5 mg liegt.

12. Zusammensetzung nach Anspruch 1 zur Verwendung nach Anspruch 8, wobei die wässrige Zusammensetzung zur Verabreichung in einem Volumen von 0,1 ml bis 25 ml vorgesehen ist.

13. Kit zum Verabreichen eines Capsaicinoids durch intraartikuläre Injektion, umfassend:
a) mindestens eine Dosiseinheit eines anästhetischen Mittels,
b) mindestens eine Dosiseinheit einer wässrigen Zusammensetzung nach einem der Ansprüche 1 bis 7,
wobei das Kit so ausgelegt ist, dass anästhetischen Mittel vor der wässrigen Zusammensetzung verabreicht wird, bevorzugt wobei das Kit ferner ein Mittel zum Verabreichen von a) und b) umfasst oder bevorzugt wobei das Kit ferner Hinweise zur Verabreichung umfasst oder bevorzugt wobei das Capsaicinoid, Hyaluronsäure und Anästhetikum zusammen formuliert werden.

## Revendications

1. Composition aqueuse pour injection chez un mammifère comprenant :
0,0002 % à 0,1 % en poids d'un capsaïcinoïde, et
99,9 % à 99,9998 % en poids d'un excipient aqueux comprenant un agent solubilisant pour solubiliser ledit capsaïcinoïde et de l'acide hyaluronique en tant qu'agent de libération prolongée pour ralentir la libération du dit capsaïcinoïde de ladite composition aqueuse lors de l'injection de ladite composition chez un mammifère,
dans laquelle ledit excipient aqueux réduit ou élimine les effets de brûlure et de picotement engendrés par ledit capsaïcinoïde lors de l'injection, et soit :
(A) la composition aqueuse n'est pas la composition de capsaïcine produite selon le procédé de l'exemple 4 de WO 2014/075084 A2, qui comprend les étapes suivantes consistant à :
(I) préparer 200 grammes d'une solution injectable de micelle à 0,06 mg/ml de capsaïcine/PS 80 de la façon suivante :
i. introduire 2 grammes de polysorbate 80 (PS 80, super-raffiné, Croda Inc., n° CAS : 9005-65-6) dans un flacon en verre pyrex de 16 ml,
ii. ajouter 0,125 grammes de trans-capsaïcine (Aversion Technologies Inc., USP 30, à 95,7 % de trans-capsaïcine, le reste étant de la cis-capsaïcine) au polysorbate 80 de l'étape (i),
iii. chauffer le mélange formé à l'étape (ii) à environ 55 °C pour dissoudre la trans-capsaïcine pour former un concentré trans-capsaïcine /PS 80,
(II) préparer une solution de capsaïcine/PS 80, PEG-300, alcool éthylique, phénol et menthol de la façon suivante :
iv. introduire 20 mg de PEG-300 (Spectrum Chemical, NF, n° CAS : 25322-68-3) dans un bécher en pyrex de 400 cm³,
v. ajouter 10 grammes d'alcool éthylique (alcool de grain de Graves, 190 Proof ) au PEG-300 et mélanger,
vi. ajouter 0,27 grammes du concentré de trans-capsaïcine/PS 80 au mélange formé à l'étape (v) et agiter,
vii. ajouter 0,2 grammes de phénol (en solution (acide phénique), Spectrum Chemical, USP, n° CAS : 108-95-2) au mélange formé à l'étape (vi),
viii. ajouter 0,2 grammes de cristaux de L-Menthol (Spectrum Chemical, USP, n° CAS : 2216-51-5) au mélange formé à l'étape (vii),
ix. chauffer le mélange formé à l'étape (viii) à environ 40 °C et laisser refroidir à température ambiante pour fournir la solution de capsaïcine/PS 80, PEG-300, alcool éthylique, phénol et menthol,
(III) préparer une solution d'acide hyaluronique de la manière suivante :
x. introduire 1 gramme d'acide hyaluronique (P.M. = 1000 kDaltons, Lotioncrafter LLC, n° CAS : 9067-32-7) dans un bécher en pyrex de 400 cm3,
xi. ajouter 169,5 grammes d'eau distillée (Poland Springs) à l'acide hyaluronique de l'étape (x) et mélanger jusqu'à l'obtention d'une solution limpide pour fournir la solution d'acide hyaluronique,
(IV) réunir la solution de capsaïcine/PS 80, PEG-300, alcool éthylique, phénol et menthol de l'étape (ix) et la solution d'acide hyaluronique de l'étape (xi) de la manière suivante :
xii. ajouter lentement la solution d'acide hyaluronique de l'étape (xi) à la solution de capsaïcine/PS 80, PEG-300, alcool éthylique, phénol et menthol de l'étape (ix) et agiter pour fournir la composition de capsaïcine produite selon le procédé de l'exemple 4 de WO 2014/075084 A2,
ou
(B) ledit agent solubilisant est du polyéthylène glycol 400 et/ou du polypropylène glycol, ou du glycérol.

2. Composition aqueuse selon la revendication 1, dans laquelle ledit capsaïcinoïde est de la capsaïcine ou de la trans-capsaïcine.

3. Composition aqueuse selon la revendication 1, dans laquelle ledit capsaïcinoïde est sous la forme d'un concentré avec un tensioactif non ionique.

4. Composition aqueuse selon la revendication 1, dans laquelle ledit agent solubilisant est du polyéthylène glycol 400 ou du polypropylène glycol.

5. Composition aqueuse selon la revendication 1, dans laquelle ledit agent de libération prolongée est de 0,5 % à 1,5 % en poids d'acide hyaluronique, ou dans laquelle ledit acide hyaluronique a un poids moléculaire moyen de 1000 kDaltons, ou dans laquelle ledit acide hyaluronique est sous la forme d'un hydrogel.

6. Composition aqueuse selon la revendication 1, comprenant en outre un tensioactif non ionique et, de préférence, soit dans laquelle le capsaïcinoïde est de la capsaïcine et le tensioactif non ionique est de l'huile de castor hydrogénée de polyoxy 40 ou dans laquelle le capsaïcinoïde est de la capsaïcine et le tensioactif non ionique est du polysorbate 80, et de préférence dans laquelle le rapport en poids de la capsaïcine au polysorbate 80 est de 1 à 5.

7. Composition aqueuse selon la revendication 1, comprenant en outre un agent anti-inflammatoire, de préférence un corticostéroïde ou comprenant en outre un agent analgésique, de préférence un ou plusieurs sélectionnés dans le groupe constitué de phénol, menthol et eugénol, ou comprenant en outre un agent anesthésique.

8. Composition selon la revendication 1 pour une utilisation dans le traitement de la douleur au niveau d'un site chez un mammifère, de préférence chez un humain, par l'administration, de préférence par injection ou par infiltration audit site chez ledit mammifère d'une quantité thérapeutiquement efficace de la composition aqueuse.

9. Composition selon la revendication 1 pour une utilisation selon la revendication 8, dans laquelle la composition est destinée à une utilisation en combinaison avec un anesthésique à administrer avant l'administration de ladite composition aqueuse, ledit agent anesthésique étant destiné de préférence à une administration dans un excipient pharmaceutiquement acceptable dans un volume de 0,1 ml à 25 ml.

10. Composition selon la revendication 1 pour une utilisation selon la revendication 8 et en outre pour une utilisation avec une application de moyens de refroidissement sur ledit site avant, pendant et/ou après l'administration de ladite composition aqueuse.

11. Composition selon la revendication 1 pour une utilisation selon la revendication 8, dans laquelle le capsaïcinoïde est de la trans-capsaïcine et le niveau de dosage va de 0,001 mg à 0,5 mg.

12. Composition selon la revendication 1 pour une utilisation cselon la revendication 8, dans laquelle ladite composition aqueuse est destinée à être administrée dans un volume de 0,1 ml à 25 ml.

13. Kit pour administrer un capsaïcinoïde par injection intra-articulaire comprenant :
a) au moins une dose unitaire d'un agent anesthésique,
b) au moins une dose unitaire d'une composition aqueuse selon l'une quelconque des revendications 1 à 7,
dans lequel ledit kit est conçu de telle manière que ledit agent anesthésique est administré avant ladite solution aqueuse, de préférence dans lequel le kit comprend en outre un moyen pour l'administration de a) et b), ou de préférence dans lequel le kit comprend en outre des instructions pour l'administration, ou de préférence dans lequel le capsaïcinoïde, l'acide hyaluronique et l'anesthésique sont formulés ensemble.
